# EUROPEAN PATENT SPECIFICATION

(11) **EP 1 233 242 B1**
(45) Date of publication and mention of the grant of the patent: **09.11.2005**
(21) Application number: 02010392.5
(22) Date of filing: 06.02.1997
(51) Int. Cl.: F25B 43/00, B01D 15/00, F24F 3/06, F24F 3/14

(54) **Air conditioner**
Klimaanlage
Dispositif de conditionnement d'air

(30) Priority: 09.02.1996 JP 2358096
(43) Date of publication of application: 21.08.2002
(62) Divisional of application: 97101886.6
(73) Proprietor: MATSUSHITA ELECTRIC INDUSTRIAL CO., LTD., Kadoma-shi, Osaka (JP)
(72) Inventor: Otaki, Shizuo, Otsu-shi, Shiga (JP)
(74) Representative: Eisenführ, Speiser & Partner

(56) References cited:
- DE-A- 3 601 342
- DE-A- 4 238 853
- DE-A- 4 319 293
- PATENT ABSTRACTS OF JAPAN vol. 015, no. 057 (M-1080), 12 February 1991 (1991-02-12) -& JP 02 287066 A (NIPPONDENSO CO LTD), 27 November 1990 (1990-11-27)
- PATENT ABSTRACTS OF JAPAN vol. 1995, no. 05, 30 June 1995 (1995-06-30) -& JP 07 043049 A (DAIKIN IND LTD), 10 February 1995 (1995-02-10)
- PATENT ABSTRACTS OF JAPAN vol. 017, no. 379 (M-1447), 16 July 1993 (1993-07-16) -& JP 05 066075 A (HITACHI LTD), 19 March 1993 (1993-03-19)

## Description

### BACKGROUND OF THE INVENTION

### (Field of the Invention)

The present invention relates to an air conditioner provided with a refrigeration cycle having a dryer.

### (Description of Related Art)

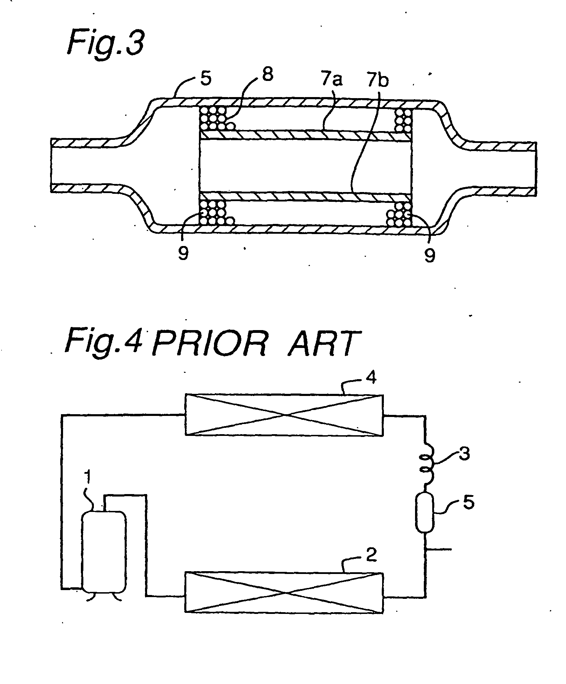

Fig, 4 depicts a conventional refrigerating cycle comprising a compressor 1, a condenser 2, a throttling device 3, an evaporator 4, and a dryer 5, all of which are connected in series. As shown in Fig. 4, the dryer 5 is interposed between the condenser 2 and the throttling device 3.

JP 05 066075 A, representing the closest prior art from which the invention proceeds, discloses a similar refigerating cycle, wherein the dryer is of a double wall construction having an external pipe and an internal pipe accommodated in the external pipe and having opposite ends being secured to the external pipe, and wherein a drying agent is accommodated in a space delimited by the external pipe and the internal pipe.

In the above-described constructions, however, when the refrigerating cycle is in operation, all refrigerant essentially passes through the drying agent accommodated in the dryer. Because of this, a refrigerant flow vibrates and wears away particles of the drying agent, and the resultant worn-out powder tends to adhere to the throttling device or the compressor, resulting in excessive throttling or poor compression.

### SUMMARY OF THE INVENTION

The present invention has been developed to overcome the above-described disadvantages.

It is accordingly an objective of the present invention to provide an air conditioner capable of preventing wear of the drying agent which has been hitherto caused by a vibration of agent particles resulting from a refrigerant flow.
In accomplishing the above and other objectives, according to the present invention, there is provided an air conditioner provided with a refrigerating cycle having a compressor, a condenser, a throttling device, an evaporator connected in series by a plurality of connecting pipes, and a dryer connected to said refrigerating cycle, wherein said dryer is connected to one of said plurality of connecting pipes and is of a double wall construction having an external pipe and an internal pipe accommodated in said external pipe, and a drying agent is accommodated in a space delimited by said external pipe and said internal pipe, characterized in that the opposite ends of said internal pipe are secured to said external pipe via associated nets, and said space accommodating said drying agent is further delimited by said nets.

Due to the construction of the present invention, the drying agent is accommodated in a space delimited by the external pipe, the internal pipe and the nets, and can effectively adsorb moisture contained in a refrigerant while preventing wear of particles of the drying agent.

### BRIEF DESCRIPTION OF THE DRAWINGS

The above and other objectives and features of the present invention will become more apparent from the following description of preferred embodiments thereof with reference to the accompanying drawings, throughout which like parts are designated by like reference numerals, and wherein:
Fig. 1 is a schematic diagram of a refrigerating cycle employed in an air conditioner according to a first explanation example which does not form part of this invention;
Fig. 2 is a diagram similar to Fig. 1, but according to a second explanation example which does not form part of this invention;
Fig. 3 is a sectional view of a dryer according to a preferred embodiment of the present invention; and
Fig. 4 is a schematic diagram of a refrigerating cycle employed in a conventional air conditioner.

### DETAILED DESCRIPTION OF THE PREFERRED EMBODIMENTS

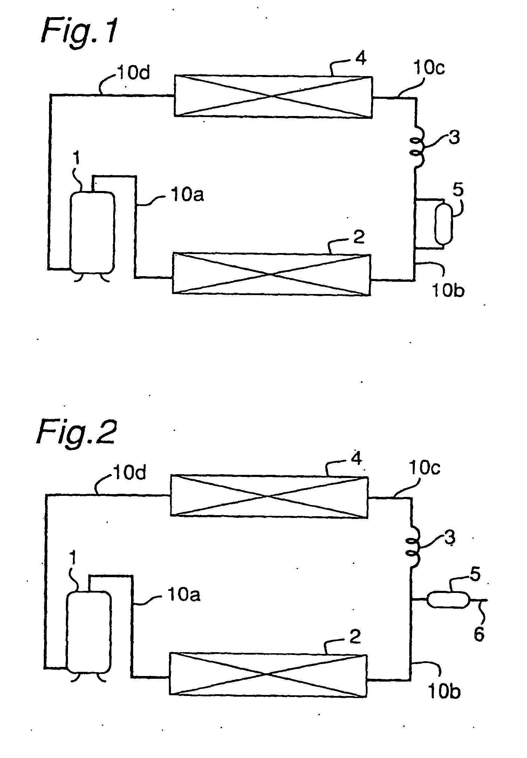

Referring now to the drawings, there is schematically shown in Fig. 1 a refrigerating cycle employed in an air conditioner according to a first explanation example being incorporated herewith for explanation purposes only which comprises a compressor 1, a condenser 2 connected to the compressor 1 via a connecting pipe 10a, a throttling device 3 connected to the condenser 2 via a connecting pipe 10b, and an evaporator 4 connected to the throttling device 3 via a connecting pipe 10c and to the compressor 1 via a connecting pipe 10d. The refrigerating cycle shown in Fig. 1 also comprises a dryer 5 connected to the connecting pipe 10b in parallel therewith.

In the refrigerating cycle of the above-described construction, when the compressor 1 is in operation, refrigerant in the compressor 1 circulates through the condenser 2, the throttling device 3, and the evaporator 4 in this order and returns to the compressor 1. Because the connecting pipe 10b connecting the condenser 2 and the throttling device 3 is a mere pipe and because the dryer 5 accommodates a drying agent, a net or meshes encircling the drying agent, and the like, most of the refrigerant flows through the connecting pipe 10b, while a small amount of refrigerant flows through the dryer 5. Because of this, the speed at which the refrigerant flows through the dryer 5 is relatively low and, hence, the refrigerant does not vibrate the drying agent in the dryer 5, thus avoiding wear of particles of the drying agent.

Fig. 2 depicts a refrigerating cycle employed in an air conditioner according to a second explanation example being incorporated here with for explanation purposes only. As shown therein, the dryer 5 is connected on its one side to the connecting pipe 10b connecting the condenser 2 and the throttling device 3 and on its other side to a filling pipe 6 through which the refrigerating cycle is charged with the refrigerant.

According to the above-described construction, the refrigerant does not pass through the dryer 5 and, hence, does not vibrate the drying agent in the dryer 5. However, because the dryer 5 communicates with the circulating refrigerant, the drying agent in the dryer 5 can capture or adsorb moisture contained in the refrigerant in the refrigerating cycle, which moisture enters the dryer 5 by diffusion regardless of whether the refrigerating cycle is in operation. Furthermore, when the refrigerant is injected into the refrigerating cycle, the refrigerant inevitably passes through the dryer 5 and, hence, the drying agent in the dryer 5 adsorbs moisture contained in the refrigerant.

Fig. 3 depicts the internal structure of a dryer 5 according to a third embodiment of the present invention. As shown therein, the dryer 5 is of a double wall construction having an external pipe 7a and an internal pipe 7b accommodated in the external pipe 7a. Opposite ends of the internal pipe 7b are secured to associated nets or meshes 9, the peripheral edges of which are in turn secured to the inner surface of the external pipe 7a. A drying agent 8 is accommodated in a space delimited by the inner surface of the external pipe 7a, the outer surface of the internal pipe 7b, and the two nets 9.

According to the above-described construction, the circulating refrigerant mainly flows through the inside of the internal pipe 7b and part thereof passes through the drying agent 8. Accordingly, even if this dryer 5 is incorporated in the conventional refrigerating cycle shown in Fig.4, the same effects as described above can be obtained.

It is to be noted here that although above the refrigerating cycle has been described as being of a unidirectional type allowing the refrigerant to flow in only one direction, similar effects can be obtained in a reversible type refrigerating cycle employing a four-way valve or the like.

## Claims

1. An air conditioner provided with a refrigerating cycle having a compressor (1), a condenser (2), a throttling device (3), an evaporator (4) connected in series by a plurality of connecting pipes (10a, 10b, 10c, 10d), and a dryer (5) connected to said refrigerating cycle;
wherein said dryer (5) is connected to one of said plurality of connecting pipes (10a, 10b, 10c, 10d) and is of a double wall construction having an external pipe (7a) and an internal pipe (7b) accommodated in said external pipe (7a); and
a drying agent (8) is accommodated in a space delimited by said external pipe (7a) and said internal pipe (7b);
**characterized in that** the opposite ends of said internal pipe (7b) are secured to said external pipe (7a) via associated nets (9); and
said space accommodating said drying agent (8) is further delimited by said nets (9).

## Patentansprüche

1. Klimaanlage, ausgestattet mit einem Kältekreislauf, welcher einen Kompressor (1), einen Kondensator (2), eine Drosselungsvorrichtung (3) und einen Verdampfer (4) aufweist, welche durch eine Mehrzahl von Verbindungsrohren (10 a, 10 b, 10 c, 10 d) in Reihe verbunden sind, und einen Trockner (5), welcher mit dem Kältekreislauf verbunden ist;
wobei der Trockner (5) mit einer der Mehrzahl von Verbindungsrohren (10 a, 10 b, 10 c, 10 d) verbunden ist und einen doppelwandigen Aufbau aufweist, der über ein äußeres Rohr (7 a) und ein inneres Rohr (7 b) verfügt, welches in dem äußeren Rohr (7 a) untergebracht ist; und
ein Trockenmittel (8) in einem Raum untergebracht ist, welcher durch das äußere Rohr (7 a) und das innere Rohr (7 b) begrenzt wird;
**dadurch gekennzeichnet, dass** die gegenüberliegenden Enden des inneren Rohres (7 b) über zugehörige Netze (9) an dem äußeren Rohr (7 a) befestigt sind; und
der Raum, welcher das Trockenmittel (8) unterbringt, ferner durch die Netze (9) begrenzt ist.

## Revendications

1. Climatiseur d'air muni d'un cycle de réfrigération ayant un compresseur (1), un condenseur (2), un dispositif d'étranglement (3), un évaporateur (4) raccordé en série par une pluralité de tuyaux de raccordement (10a, 10b, 10c, 10d), et un dispositif de séchage (5) raccordé audit cycle de réfrigération ;
dans lequel ledit dispositif de séchage (5) est raccordé à un de ladite pluralité de tuyaux de raccordement (loa, 10b, 10c, 10d) et est d'une structure à double paroi ayant un tuyau externe (7a) et un tuyau interne (7b) reçu dans ledit tuyau externe (7a) ; et un agent de séchage (8) est reçu dans un espace délimité par ledit tuyau externe (7a) et ledit tuyau interne (7b) ;
**caractérisé en ce que** les extrémités opposées dudit tuyau interne (7b) sont fixées audit tuyau externe (7a) par des réseaux associés (9) ; et
ledit espace recevant ledit agent de séchage (8) est de plus délimité par lesdits réseaux (9).
